(19) Europäisches Patentamt
European Patent Office
Office européen des brevets

(11) EP 3 736 263 A1

(12) EUROPÄISCHE PATENTANMELDUNG

(43) Veröffentlichungstag:
**11.11.2020 Patentblatt 2020/46**

(51) Int Cl.:
***C07C 263/20*** *(2006.01)* ***C07C 265/14*** *(2006.01)*

(21) Anmeldenummer: **19172870.8**

(22) Anmeldetag: **07.05.2019**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **Covestro Deutschland AG**
**51373 Leverkusen (DE)**

(72) Erfinder: **Die Erfindernennung liegt noch nicht vor**

(74) Vertreter: **Levpat**
**c/o Covestro AG**
**Gebäude 4825**
**51365 Leverkusen (DE)**

# (54) VERFAHREN ZUR REINIGUNG VON ISOCYANATEN

(57) Die Erfindung betrifft ein Verfahren zur Herstellung und Reinigung mindestens eines Isocyanates ausgewählt aus der Gruppe bestehend aus aliphatischen, cycloaliphatischen, araliphatischen Isocyanaten und Mischungen davon.

Das Verfahren umfasst entweder einen Filtrationsschritt mit einem Filter, der eine Permeabilität von 5 bis 100 l/(m2 x min) hat, oder aber zwei Filtrationsschritte, bei denen der erste Filter eine Permeabilität von 5 bis 1000 l/(m2 x min) hat und der zweite Filter eine maximale Porengrösse von 0,02 bis 10 micrometer aufweist.

EP 3 736 263 A1

**Beschreibung**

Die Erfindung betrifft ein Verfahren zur Reinigung mindestens eines Isocyanates ausgewählt aus der Gruppe bestehend aus aliphatischen, cycloaliphatischen, araliphatischen Isocyanaten und Mischungen davon, ein Verfahren zur Herstellung mindestens eines Isocyanates ausgewählt aus der Gruppe bestehend aus aliphatischen, cycloaliphatischen, araliphatischen Isocyanaten und Mischungen davon, das Isocyanat, erhältlich durch dieses Verfahren, ein Polyurethan, aufgebaut aus mindestens einem solchen Isocyanat, die Verwendung dieses Isocyanates und ein optisches Bauteil, enthaltend mindestens ein erfindungsgemäßes Polyurethan.

Aliphatische, cycloaliphatische oder araliphatische Diisocyanate werden üblicherweise durch Phosgenierung der entsprechenden Diamine hergestellt. Unabhängig von der genauen Verfahrensweise fällt das Reaktionsprodukt dabei üblicherweise als Gemisch aus monomeren und polymeren Spezies an. Dieses rohe Verfahrensprodukt wird in einer dem Fachmann bekannten Weise aufgearbeitet, wobei die Aufarbeitung im Allgemeinen eine mehrstufige VakuumDestillation umfasst und das monomere Diisocyanat in hoher Reinheit liefert. Solche Diisocyanate sind für die Herstellung von lichtechten, d.h. nicht vergilbenden, Polyurethanen geeignet.

Sollen optische Materialien, wie beispielsweise optische Linsen, hergestellt werden, so reicht es in der Regel nicht aus, lichtechte Polyurethane zu verwenden. Es werden darüber hinaus erhöhte Anforderungen an die Klarheit des Materials gestellt, d.h. die Trübungswerte müssen sehr niedrig sein.

Die DE 1805957 sowie die US 3,658,656 beschreiben beispielsweise Verfahren zur Reinigung von Xylylendiisocyanat (XDI), welches aus Xylylendiamin (XDA) und Phosgen hergestellt wurde. Dazu wird das aus der Reaktion erhaltene XDI zunächst in Gegenwart eines inerten Gases unter Vakuum destilliert, um leichtsiedende Verunreinigungen abzutrennen. Im Sumpf der Destillationsvorrichtung reichert sich dabei XDI zusammen mit höhermolekularem Rückstand an. In einer weiteren Destillationsstufe wird dieses XDI aus dem Sumpfprodukt als Destillat abgetrennt. Die Destillation erfolgt dabei beispielsweise bei einem Druck von 5 bis 200 mmHg am Kolonnenkopf und einer Temperatur von 170 bis 185 °C im Kolonnensumpf.

Während die auf diese Weise ausgehend von aliphatischen, cycloaliphatischen oder araliphatischen Aminen hergestellten, lichtechten Diisocyanate zwar eine hohe Reinheit, z.B. in Bezug auf chlorierte Nebenprodukte aufweisen, wurde jedoch festgestellt, dass sie zu Trübungen neigen und die diesbezüglichen Anforderungen für die Verwendung in optischen Materialien nicht immer erfüllen.

Möglicherweise kommt es trotz der geringeren Reaktivität der aliphatisch, cycloaliphatisch oder araliphatisch gebundenen NCO-Gruppen im Vergleich zu aromatisch gebundenen NCO-Gruppen überraschend zu einer Di- oder Oligomerisierung der Diisocyanate. Für die sehr reaktiven aromatischen Diphenylmethandiisocyanate ist beispielsweise in der GB 1413074 die Bildung von Uretdion-Dimeren im Destillat beschrieben, die sich durch ein schnelles Abkühlen des Destillats weitgehend vermeiden lässt. Der Versuch, diese technische Lehre beispielsweise auf Xylylendiisocyanat zu übertragen, zeigte jedoch, dass sich Trübungen auf diese Art nicht hinreichend vermeiden lassen.

Die WO 2007/051740 A1 beschreibt ein Verfahren zur Herstellung von Gemischen enthaltend Diphenylmethandiisocyanat und dessen höhermolekulare Homologe. Es wird beschrieben, dass aus einem Diphenylmethandiisocyanat (2-Kern-MDI) und Triphenylmethandiisocyanat (3-Kern-MDI) enthaltenden Polyisocyanat-Gemisch bei Raumtemperatur ein Zwei-Phasen-Gemisch mit einer flüssigen und einer festen kristallinen Phase entsteht. Aus diesem kann durch einfaches Dekantieren oder Filtrieren die flüssige Phase abgetrennt werden, um das Verhältnis von 2-Kernzu 3-Kern-MDI einzustellen. Auf die Bedingungen der möglichen Filtration wird nicht weiter eingegangen und aufgrund der verwendeten Diisocyanate wird weder ein lichtechtes, noch ein trübungsfreies Diisocyanat erhalten.

Ausgehend von diesem Stand der Technik lag der vorliegenden Erfindung die Aufgabe zu Grunde, mindestens einen, vorzugsweise mehrere, der oben genannten Nachteile des Standes der Technik zu beheben. Insbesondere lag der vorliegenden Erfindung die Aufgabe zu Grunde, ein Verfahren zur Aufreinigung mindestens eines aliphatischen, cycloaliphatischen oder araliphatischen Diisocyanates bereitzustellen, mit dem es möglich ist, die Trübung des entsprechenden Diisocyanates auf einen Wert einzustellen, so dass Polyurethane erhalten werden, die in optischen Anwendungen, insbesondere als optische Linsen, eingesetzt werden können. Des Weiteren ist es eine Aufgabe der vorliegenden Erfindung ein Verfahren bereitzustellen, mit dem die genannten Diisocyanate in entsprechend hoher optischer Qualität, insbesondere mit einer sehr geringen Trübung, erhalten werden können.

Diese Aufgaben werden gelöst durch das erfindungsgemäße Verfahren zur Reinigung mindestens eines Isocyanates ausgewählt aus der Gruppe bestehend aus aliphatischen, cycloaliphatischen, araliphatischen Isocyanaten und Mischungen davon, umfassend mindestens die folgenden Schritte:

(A) Bereitstellen des mindestens einen Isocyanates,

(B1) Filtrieren des mindestens einen Isocyanates aus Schritt (A) über einen Filter, der eine Permeabilität von 5 bis 100 l/($m^2 \cdot$ min) (bei Standardbedingungen, $\Delta p$ = 1 bar, $H_2O$, 20 °C) aufweist, um das mindestens eine gereinigte Isocyanat zu erhalten,

oder umfassend mindestens die folgenden Schritte:

(A) Bereitstellen des mindestens einen Isocyanates,

(B2) Filtrieren des mindestens einen Isocyanates aus Schritt (A) über einen Filter, der eine Permeabilität von 5 bis 1000 l/($m^2$ · min) (bei Standardbedingungen, $\Delta p$ = 1 bar, $H_2O$, 20 °C) aufweist, und

(C) Filtrieren des mindestens einen Isocyanates über einen Filter mit einer maximalen Porengröße von 0,02 bis 10 $\mu$m,

wobei die Reihenfolge der Schritte (A), (B2), (C) oder (A), (C), (B2) sein kann, um das mindestens eine gereinigte Isocyanat zu erhalten.

Die einzelnen Schritte des erfindungsgemäßen Verfahrens werden im Folgenden detailliert beschrieben.

Schritt (A) des erfindungsgemäßen Verfahrens umfasst das Bereitstellen des mindestens einen Isocyanates.

Erfindungsgemäß kann in Schritt (A) jedes dem Fachmann bekannte Isocyanat ausgewählt aus der Gruppe bestehend aus aliphatischen, cycloaliphatischen, araliphatischen Isocyanaten und Mischungen davon eingesetzt werden. Insbesondere ist das erfindungsgemäß eingesetzte Isocyanat ausgewählt aus der Gruppe der aliphatischen, cycloaliphatischen, araliphatischen Diisocyanate und Mischungen davon.

Erfindungsgemäß besonders bevorzugt eingesetzte aliphatische Diisocyanate sind beispielsweise ausgewählt aus der Gruppe bestehend aus Hexamethylendiisocyanat (HDI), Pentamethylendiisocyanat (PDI), 1,4-Butandiisocyanat, 1,8-Diisocyanatooctan, 1,9-Diisocyanatononan, 1,10-Diisocyanatodecan, 1,11-Diisocyanatoundecan, 1,12-Diisocyanatododecan, 2-Methylpentamethylendiisocyanat, 2,2-Dimethylpentamethylendiisocyanat, Neopentandiisocyanat, 2,2,4-Trimethylhexamethylendiisocyanat und 2,4,4-Trimethylhexamethylendiisocyanat und Mischungen davon. Ganz besonders bevorzugt eingesetzte aliphatische Diisocyanate sind ausgewählt aus der Gruppe bestehend aus HDI und PDI.

Erfindungsgemäß besonders bevorzugt eingesetzte cycloaliphatische Diisocyanate sind beispielsweise ausgewählt aus der Gruppe bestehend aus 3-Isocyanatmethyl-3,5,5-trimethylcyclohexylisocyanat (IPDI), 2,4'-Diisocyanatodicyclohexylmethan (2,4'-H12-MDI), 4,4'-Diisocyanatodicyclohexylmethan (4,4'-H12-MDI), 2,4'-Methylen-bis(cyclohexyl)diisocyanat, 4,4'-Diisocyanato-3,3'-dimethyldicyclohexylmethan, 2,4- und 2,6-Diisocyanato-methyl-cyclohexan (H6TDI), 1,3-Cyclohexandiisocyanat, 1,4-Cyclohexandiisocyanat, 1,3-Diisocyanato-2-methylcyclohexan, 1,3-Diisocyanato-4-methyl-cyclohexan, 1,4-Diisocyanato-3,3,5-trimethylcyclohexan, 1,3-Bis(isocyanatomethyl)cyclohexan (H6XDI), 1,4-Bis(isocyanatomethyl)cyclohexan, den Isomeren des Bis(isocyanatomethyl)bicyclo[2.2.1]heptans (NBDI), insbesondere 2,5-Bis(isocyanatomethyl)bicyclo[2.2.1]heptan (2,5-NBDI) oder 2,6-Bis(isocyanatomethyl)bicyclo[2.2.1]heptan (2,6-NBDI), und Mischungen davon.

Erfindungsgemäß besonders bevorzugt eingesetzte araliphatische Diisocyanate sind beispielsweise ausgewählt aus der Gruppe bestehend aus Xylylendiisocyanat (XDI), insbesondere 1,3-Xylylendiisocyanat (m-XDI) oder 1,4-Xylylendiisocyanat (p-XDI), 1,3-Pis(1-isocyanato-1-methylethyl)benzol (m-TMXDI), 1,4-Bis(1-isocyanato-1-methylethyl)benzol (p-TMXDI). und Mischungen davon. Ganz besonders bevorzugt ist meta-Xylylendiisocyanat. Meta-Xylyendiisocyanat (1) und para-Xylylendiisocyanat (2) sind im Folgenden dargestellt:

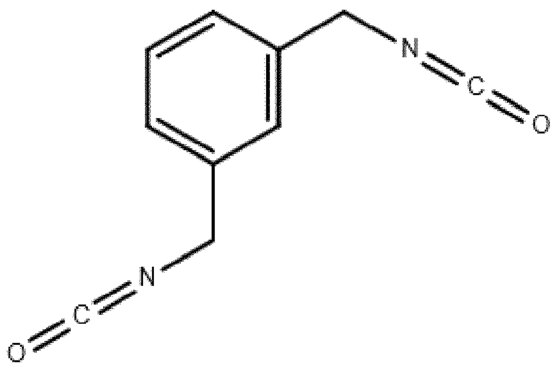

(1)

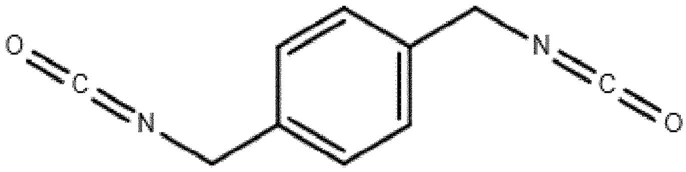

(2)

Ganz besonders bevorzugt ist in Schritt (A) des erfindungsgemäßen Verfahrens das Isocyanat ausgewählt aus der Gruppe bestehend aus Bis(isocyanatomethyl)bicyclo[2.2.1]heptan (NBDI), insbesondere 2,5-Bis(isocyanatomethyl)bicyclo[2.2.1]heptan (2,5-NBDI) oder 2,6-Bis(isocyanatomethyl)bicyclo[2.2.1]heptan (2,6-NBDI), Pentamethylendiisocyanat, 1,3-Bis(isocyanatomethyl)cyclohexan (H6XDI), Xylylendiisocyanat, insbesondere meta-Xylylendiisocyanat und/oder para-Xylylendiisocyanat, und Mischungen davon.

Das Bereitstellen des mindestens einen Isocyanates, insbesondere des mindestens einen Diisocyanates, in Schritt (A) des erfindungsgemäßen Verfahrens umfasst insbesondere das Herstellen des mindestens einen Isocyanates und bevorzugt auch eine erste Aufreinigung, um das Produkt zu erhalten, welches dann in den Stufen (B1) oder (B2) und (C) erfindungsgemäß aufgereinigt wird.

Insbesondere umfasst Schritt (A) des erfindungsgemäßen Verfahrens die Herstellung des mindestens einen Isocyanates durch Phosgenierung des entsprechenden mindestens einen Amins mit Phosgen.

Für den besonders bevorzugten Fall, dass erfindungsgemäß Bis(isocyanatomethyl)bicyclo[2.2.1]heptan (NBDI), insbesondere 2,5-Bis(isocyanatomethyl)bicyclo[2.2.1]heptan (2,5-NBDI) oder 2,6-Bis(isocyanatomethyl)bicyclo[2.2.1]heptan (2,6-NBDI), Pentamethylendiisocyanat, 1,3-Bis(isocyanatomethyl)cyclohexan (H6XDI), Xylylendiisocyanat, insbesondere meta-Xylylendiisocyanat und/oder para-Xylylendiisocyanat, oder Mischungen davon aufgereinigt bzw. hergestellt werden, werden in der Phosgenierung bevorzugt Bis(aminomethyl)bicyclo[2.2.1]heptan (NBDA), insbesondere 2,5-Bis(aminomethyl)bicyclo[2.2.1]heptan (2,5-NBDA) oder 2,6-Bis(aminomethyl)bicyclo[2.2.1]heptan (2,6-NBDA), Pentamethylendiamin, 1,3-Bis(aminomethyl)cyclohexan (H6XDA), Xylylendiamin, insbesondere meta-Xylylendiamin und/oder para-Xylylendiamin, oder Mischungen davon als Ausgangsamine eingesetzt.

Für den ganz besonders bevorzugten Fall, dass erfindungsgemäß Xylylendiisocyanat, insbesondere m-Xylylendiisocyanat aufgereinigt bzw. hergestellt wird, wird in der Phosgenierung bevorzugt Xylylendiamin, insbesondere m-Xylylendiamin als Ausgangsamin eingesetzt.

Das Herstellen des mindestens einen Isocyanates durch Phosgenierung des mindestens einen Amins kann auf verschiedene Weise erfolgen.

In einer Ausführungsform erfolgt die Phosgenierung des mindestens einen Amins in der Gasphase. Dazu wird das Amin bevorzugt verdampft und auf eine Temperatur innerhalb des Temperaturbereichs von 200 bis 600 °C erhitzt. Optional erfolgt die Verdampfung und auch der Einsatz der bei der Verdampfung erzeugten Amin-Dämpfe in Gegenwart eines Inertgases und/oder von Dämpfen eines inerten Lösungsmittels. Bevorzugtes Inertgas ist Stickstoff. Geeignete inerte Lösungsmittel sind beispielsweise Chlorbenzol, o-Dichlorbenzol, Xylol, Chlornaphthalin oder deren Gemische.

Das bei der Phosgenierung verwendete Phosgen wird, bezogen auf das Amin, bevorzugt im Überschuss eingesetzt. Im Allgemeinen genügt eine Phosgenmenge, die 150 bis 350% der Theorie bezüglich der ablaufenden Phosgenierungsreaktion entspricht. Der Phosgenstrom wird vor der Reaktion bevorzugt auf eine Temperatur innerhalb des Bereichs von 200 bis 600 °C erhitzt.

Zur Durchführung der Phosgenierung werden der vorerhitzte, amin-haltige Strom und der ebenfalls vorerhitzte Phosgenstrom bevorzugt kontinuierlich in einen zylindrischen Reaktionsraum geleitet und dort miteinander vermischt. Geeignete zylindrische Reaktionsräume sind beispielsweise Rohrreaktoren, welche im Allgemeinen aus Stahl, Glas, legiertem oder emailliertem Stahl bestehen. Sie weisen im Allgemeinen eine Länge auf, die ausreichend ist, um unter den Verfahrensbedingungen eine vollständige Umsetzung des Amins mit dem Phosgen zu ermöglichen. Des Weiteren sind die Abmessungen des Reaktionsraums bevorzugt so gewählt, dass im Reaktionsraum eine turbulente Strömung mit einer Reynoldszahl von mindestens 2500 vorherrscht. Dies ist im Allgemeinen dann gewährleistet, wenn die Strömungsgeschwindigkeit mehr als 90 m/s beträgt. Eine solche Strömungsgeschwindigkeit kann durch Einstellen eines entsprechenden Differenzdrucks zwischen den Produktleitungen zum Reaktionsraum und dem Ausgang aus dem Reaktionsraum sichergestellt werden. Im Allgemeinen liegt der Druck in den Zuleitungen bei 200 bis 300 mbar(ü) und am Ausgang aus dem Reaktionsraum bei 150 bis 200 mbar(ü).

Nach der erfolgten Phosgenierungsreaktion im Reaktionsraum wird das den Reaktionsraum kontinuierlich verlassende Gemisch bevorzugt von dem gebildeten Isocyanat befreit. Dies kann beispielsweise durch selektive Kondensation in einem inerten Lösungsmittel wie beispielswiese Chlorbenzol oder Dichlorbenzol erfolgen. Sofern der aminhaltige Strom bereits ein inertes Lösungsmittel enthielt, ist es bevorzugt, hier das gleiche Lösungsmittel zu verwenden.

Die Temperatur des Lösungsmittels wird dabei bevorzugt so gewählt, dass sie einerseits oberhalb der Zersetzungstemperatur des dem Isocyanat entsprechenden Carbamidsäurechlorids liegt und anderseits das Isocyanat kondensiert bzw. sich in dem Lösungsmittel löst, während Phosgen, Chlorwasserstoff und gegebenenfalls Inertgas die Kondensationsstufe gasförmig durchlaufen. Besonders geeignet sind Lösungsmitteltemperaturen im Bereich von 120 bis 200 °C.

Das die Kondensationsstufe zur Gewinnung des mindestens einen Isocyanates durchlaufende Gasgemisch wird anschließend bevorzugt in an sich bekannter Weise von überschüssigem Phosgen befreit. Dies kann mittels einer Kühlfalle, Absorption in einem bei einer Temperatur von -10 °C bis 8 °C gehaltenen inerten Lösungsmittel (z.B. Chlorbenzol, MCB, oder Dichlorbenzol, ODB) oder durch Adsorption und Hydrolyse an Aktivkohle erfolgen. Das die Phosgen-Rückgewinnungsstufe durchlaufende Chlorwasserstoffgas kann bevorzugt in an sich bekannter Weise zur Rückgewinnung des zur Phosgensynthese erforderlichen Chlors recykliert werden.

In einer weiteren Ausführungsform erfolgt die Phosgenierung des mindestens einen Amins in der flüssigen Phase. Die Reaktion kann dann auf verschiedene Weise ausgeführt werden. Entweder wird das Amin direkt mit Phosgen in einem inerten flüssigen Medium umgesetzt (Basenphosgenierung) oder das Amin wird zunächst durch Umsetzung mit Chlorwasserstoffgas oder Kohlendioxid in einem inerten flüssigen Medium das entsprechende Salz überführt und dann mit Phosgen umgesetzt (Hydrochlorid- bzw. Carbaminatphosgenierung). Als flüssiges Medium eigenen sich für alle Phosgenierungen insbesondere Chlorbenzol und/oder Dichlorbenzol.

Bei der Basenphosgenierung wird die Reaktion zweistufig in dem inerten flüssigen Medium durchgeführt. Solche Reaktionen sind beispielsweise in W. Siefken, Liebigs Annalen der Chemie, 562 (1949) S. 96 beschrieben. Im ersten Stadium, der Kalt-Phosgenierung, wird die Temperatur der Reaktionsmischung bevorzugt in einem Bereich zwischen 0 und 100 °C gehalten. Es entsteht eine Suspension, die Carbaminsäurechlorid, Aminhydrochlorid und geringe Mengen an freiem Isocyanat enthält. Bevorzugt wird dabei eine Lösung von Phosgen in einem inerten Lösungsmittel vorgelegt und dann eine Lösung oder Suspension des Amins im gleichen Lösungsmittel sowie gegebenenfalls weiteres Phosgen zugegeben. Auf diese Weise wird die Konzentration von freiem Amin gering gehalten und somit die unerwünschte Bildung von Harnstoffen unterdrückt.

Im zweiten Stadium, der Heiß-Phosgenierung, wird die Temperatur erhöht und liegt vorzugsweise in einem Bereich von 120 bis 200 °C. Sie wird in diesem Bereich gehalten, während so lange weiteres Phosgen zugeführt wird, bis die Umsetzung zum Isocyanat beendet ist, also die HCl-Entwicklung zum Erliegen kommt. Phosgen wird dabei zweckmäßig im Überschuss verwendet. Bei Bedarf kann die Reaktion sowohl in der Kalt- als auch in der Heißphosgenierung unter Einleitung eines inerten Gases durchgeführt werden.

Bei der Amin-Hydrochlorid- oder Carbaminat-Phosgenierung wird das Amin bevorzugt zunächst mit Chlorwasserstoffgas oder Kohlendioxid in einem inerten flüssigen Medium zur Erzeugung des entsprechenden Salzes umgesetzt. Die Reaktionstemperatur liegt während dieser Salzbildung vorzugsweise in einem Bereich von 0 bis 80 °C. Es schließt sich der Phosgenierschritt als zweiter Schritt an, der im Wesentlichen der Heiß-Phosgenierung aus der oben beschriebenen Basenphosgenierung ähnelt. Es wird also auch hier die Temperatur bevorzugt im Bereich von 120 bis 200 °C gehalten während Phosgen und optional ein Inertgas in die Reaktionsmischung eingeleitet werden. Die Einleitung erfolgt so lange, bis die Umsetzung zum Isocyanat beendet ist. Auch hier wird Phosgen vorzugsweise im Überschuss eingesetzt, um die Reaktion zu beschleunigen.

Sowohl bei der Basenphosgenierung als auch bei der Amin-Hydrochlorid- bzw. der Carbaminat-Phosgenierung wird nach Beendigung der Reaktion das verbliebene Phosgen und Chlorwasserstoffgas bevorzugt mit einem Inertgas, vorzugsweise mit Sticktstoff, ausgeblasen. Bei Bedarf kann eine Filtration erfolgen, um gegebenenfalls vorhandene Feststoffe wie unreagierte Amin-Hydrochloride zu entfernen.

Das so erhaltene mindestens eine Isocyanat wird bevorzugt destillativ aufgearbeitet, bevor es erfindungsgemäß in den Schritten (B1) oder (B2) weiterbehandelt wird. Insbesondere wird bei dieser destillativen Aufarbeitung das mindestens eine Isocyanat von dem eingesetzten Lösungsmittel, chlorierten Nebenprodukten und höhersiedenden Rückständen befreit.

Die vorliegende Erfindung betrifft daher insbesondere das erfindungsgemäße Verfahren, wobei Schritt (A) das Destillieren des mindestens einen Isocyanates, insbesondere vor den Schritten (B1) oder (B2), umfasst.

Das in Schritt (A) des erfindungsgemäßen Verfahrens bevorzugt durchgeführte Destillieren kann auf dem Fachmann bekannte Art und Weise erfolgen. Da zur Herstellung der Isocyanate üblicherweise Lösungsmittel verwendet werden, die einen niedrigeren Siedepunkt als das jeweilige Isocyanat aufweisen, umfasst die Destillation regelmäßig eine Lösungsmittelabtrennung. In diesem Destillationsschritt können auch leichtsiedende Nebenkomponenten, insbesondere chlorierte leichtsiedende Nebenkomponenten abgetrennt werden. Das Lösungsmittel kann, gegebenenfalls nach weiteren Aufreinigungsschritten, wieder in der Reaktion zur Herstellung des Isocyanates, bevorzugt Diisocyanates, eingesetzt werden.

Außerdem umfasst ein solches Reinigungsverfahren eine Reindestillation zur Abtrennung des Isocyanates von schwersiedendem Rückstand.

Alle Destillationsschritte erfolgen bevorzugt im Vakuum, um die erforderlichen Temperaturen für die Destillation und damit die thermische Belastung des Produkts zu verringern. Insbesondere werden die Destillationsschritte bei

Drücken von 1 bis 500 mbar (a) und einer Sumpf-Temperatur von 90 bis 250 °C, bevorzugt 120 bis 190 °C, besonders bevorzugt 120 bis 170 °C durchgeführt. Insbesondere bei der Reindestillation liegt der Druck bevorzugt im Bereich von 1 bis 100 mbar(a), besonders bevorzugt im Bereich von 5 bis 50 mbar(a) und die Sumpftemperatur im Bereich von 120 bis 185 °C, ganz besonders bevorzugt im Bereich von 120 bis 170 °C.

Um bei Bedarf die Bildung von Uretdionen im Destillat zurückzudrängen, erfolgt die Abkühlung des Destillates möglichst rasch.

Nach Schritt (A) des erfindungsgemäßen Verfahrens wird bevorzugt das mindestens eine Isocyanat insbesondere meta-Xylylendiisocyanat und/oder para-Xylylendiisocyanat, in reiner Form, d.h. in einer Reinheit von ≥ 99,0%, bevorzugt von ≥ 99,5% und besonders bevorzugt von ≥ 99,7% erhalten.

Das in Schritt (A) erhaltene mindestens eine Isocyanat weist allerdings eine Trübung auf, die beispielsweise bei größer 0,5 NTU, bevorzugt 0,5 bis 40 NTU, jeweils bestimmt nach DIN EN ISO 7027-1:2016-11, liegt. Das erfindungsgemäß in Schritt (A) bereitgestellte mindestens eine Isocyanat kann ohne weitere Zwischenschritte in Schritt (B1) oder (B2) bzw. (C) überführt werden.

In einer bevorzugten Ausführungsform wird das Isocyanat jedoch nach Schritt (A), bevorzugt vor Schritt (B1) oder (B2), einer Reifung unterzogen. Hierzu wird das Isocyanat bevorzugt bei einer Temperatur im Bereich von 0 bis 100 °C, besonders bevorzugt im Bereich von 10 bis 80 °C, ganz besonders bevorzugt im Bereich von 15 bis 55 °C, gelagert. Optional kann das Isocyanat während der Lagerung gerührt werden, um das Absetzen von eventuell auftretenden Feststoffen am Boden des Lagerbehälters zu vermeiden. Bevorzugt erfolgt die Reifung über einen Zeitraum von 1 bis 500 h, besonders bevorzugt über einen Zeitraum von 2 bis 100 h. Auf diese Weise können im Isocyanat gelöste Vorläuferstoffe, also Stoffe, die zur Bildung von Trübungen im Isocyanat beitragen können, gebunden und somit besser im anschließenden Filtrationsschritt entfernt werden. Eine solche Vorgehensweise wirkt sich vorteilhaft auf die Lagerfähigkeit des Isocyanates aus.

Erfindungsgemäß kann das in Schritt (A) bereitgestellte mindestens eine Isocyanat in einer ersten Alternative in Schritt (B1) überführt und filtriert werden, d.h. die Reihenfolge der Schritte für diese Ausführungsform lautet (A), gefolgt von (B1). In einer zweiten Ausführungsform kann das in Schritt (A) bereitgestellte mindestens eine Isocyanat in die Schritte (B2) und (C) überführt und filtriert werden, d.h. die Reihenfolge der Schritte für diese Ausführungsform lautet (A), gefolgt von (B2), gefolgt von (C) oder (A), gefolgt von (C), gefolgt von (B2), bevorzugt (A), gefolgt von (B2), gefolgt von (C). Gemäß allen erfindungsgemäßen Ausführungsformen wird das mindestens eine Isocyanat mit den erfindungsgemäßen Vorteilen, d.h. insbesondere einer niedrigen Trübung von weniger als 0,35 NTU, erhalten.

Schritt (B1) des erfindungsgemäßen Verfahrens umfasst das Filtrieren des mindestens einen Isocyanates aus Schritt (A) über einen Filter, der eine Permeabilität von 5 bis 100 1/($m^2$ · min) (bei Standardbedingungen, $\Delta p$ = 1 bar, $H_2O$, 20 °C) aufweist, um das mindestens eine gereinigte Isocyanat zu erhalten.

In Schritt (B1) des erfindungsgemäßen Verfahrens wird das in Schritt (A) erhaltene mindestens eine Isocyanat über einen Filter mit einer Permeabilität von 5 bis 100 1/($m^2$ · min), bevorzugt 5 bis 60 1/($m^2$ · min), besonders bevorzugt 5 bis 40 1/($m^2$ · min), jeweils bei Standardbedingungen, $\Delta p$ = 1 bar, $H_2O$, 20 °C, filtriert.

Erfindungsgemäß kann Schritt (B1) bei jeder dem Fachmann als geeignet erscheinenden Temperatur erfolgen. Bevorzugt wird Schritt (B1) bei einer Temperatur von 5 bis 190 °C, besonders bevorzugt 5 bis 100 °C, ganz besonders bevorzugt 5 bis 60 °C, durchgeführt.

Erfindungsgemäß kann Schritt (B1) bei jedem dem Fachmann als geeignet erscheinenden Druck erfolgen. Bevorzugt wird Schritt (B1) bei einem Druck von 0,1 bis 7 bar(a), besonders bevorzugt 0.5 bis 6 bar(a), ganz besonders bevorzugt 1 bis 4 bar(a), jeweils gemessen auf der Anströmseite des Filters, durchgeführt.

Abgesehen von der oben genannten Permeabilität unterliegt der in Schritt (B1) des erfindungsgemäßen Verfahrens eingesetzte Filter keinen weiteren Einschränkungen. Bevorzugt enthält der in Schritt (B1) eingesetzte Filter mindestens ein Material ausgewählt aus Naturfasern, synthetischen Polymeren, Perlit, Kieselgur und Mischungen davon. Weiter bevorzugt wird erfindungsgemäß eine Filterschicht aus dem genannten Material eingesetzt. Geeignete Vorrichtungen zur Aufnahme der der entsprechenden Filterschicht sind dem Fachmann an sich bekannt.

Weiter bevorzugt liegt in Schritt (B1) des erfindungsgemäßen Verfahrens ein spezifischer Filtratfluss, von 50 bis 1500 kg/($m^2$·h), bevorzugt 100 bis 1000 kg/($m^2$·h), besonders bevorzugt 200 bis 500 kg/($m^2$·h), vor.

Ebenfalls weiter bevorzugt liegt in Schritt (B1) der Druckverlust über den Filter bei 0,01 bis 5 bar, bevorzugt bei 0,1 bis 3 bar und besonders bevorzugt bei 0,5 bis 2 bar, ganz besonders bevorzugt bei 1 bis 1,5 bar.

Nach Schritt (B1) des erfindungsgemäßen Verfahrens weist das mindestens eine gereinigte Isocyanat, bevorzugt Xylylendiisocyanat, insbesondere bevorzugt meta-Xylylendiisocyanat und/oder para-Xylylendiisocyanat, eine Trübung von weniger als 0,35 NTU, besonders bevorzugt 0,05 bis 0,35 NTU, ganz besonders bevorzugt 0,10 bis 0,32 NTU, jeweils bestimmt nach DIN EN ISO 7027-1:2016) auf.

Durch die durch das erfindungsgemäße Verfahren umfassend die Schritte (A) und (B1) erhaltene besonders niedrige Trübung kann das mindestens eine gereinigte Isocyanat bevorzugt zur Synthese von Polyurethanen für optische Anwendungen eingesetzt werden.

Gemäß einer zweiten Ausführungsform des erfindungsgemäßen Verfahrens wird das in Schritt (A) bereitgestellte mindestens eine Isocyanat in Schritt (B2) überführt.

Schritt (B2) des erfindungsgemäßen Verfahren betrifft das Filtrieren des mindestens einen Isocyanates aus Schritt (A) über einen Filter, der eine Permeabilität von 5 bis 1000 1/($m^2$ · min), bei Standardbedingungen, $\Delta p$ = 1 bar, $H_2O$, 20 °C, aufweist.

In Schritt (B2) des erfindungsgemäßen Verfahrens wird das in Schritt (A) erhaltene mindestens eine Isocyanat über einen Filter mit einer Permeabilität von 5 bis 1000 1/($m^2$ · min), bevorzugt 5 bis 950 1/($m^2$ · min), besonders bevorzugt 80 bis 980 1/($m^2$ · min), jeweils bei Standardbedingungen, $\Delta p$ = 1 bar, $H_2O$, 20 °C, filtriert.

Erfindungsgemäß kann Schritt (B2) bei jeder dem Fachmann als geeignet erscheinenden Temperatur erfolgen. Bevorzugt wird Schritt (B2) bei einer Temperatur von 5 bis 190 °C, besonders bevorzugt 5 bis 100 °C, ganz besonders bevorzugt 5 bis 60 °C, durchgeführt.

Erfindungsgemäß kann Schritt (B2) bei jedem dem Fachmann als geeignet erscheinenden Druck erfolgen. Bevorzugt wird Schritt (B2) bei einem Druck von 0,1 bis 7 bar(a), besonders bevorzugt 0.5 bis 6 bar(a), ganz besonders bevorzugt 1 bis 4 bar(a), jeweils gemessen auf der Anströmseite des Filters, durchgeführt.

Abgesehen von der oben genannten Permeabilität unterliegt der in Schritt (B2) des erfindungsgemäßen Verfahrens eingesetzte Filter keinen weiteren Einschränkungen. Bevorzugt enthält der in Schritt (B2) eingesetzte Filter mindestens ein Material ausgewählt aus Naturfasern, synthetischen Polymeren, Perlit, Kieselgur und Mischungen davon. Weiter bevorzugt wird erfindungsgemäß eine Filterschicht aus dem genannten Material eingesetzt. Geeignete Vorrichtungen zur Aufnahme der der entsprechenden Filterschicht sind dem Fachmann an sich bekannt, beispielsweise Modulfilter.

Weiter bevorzugt liegt in Schritt (B2) des erfindungsgemäßen Verfahrens ein spezifischer Filtratfluss, von 50 bis 1500 kg/($m^2$·h), bevorzugt 100 bis 1000 kg/($m^2$·h), besonders bevorzugt 500 bis 800 kg/($m^2$·h) vor.

Ebenfalls weiter bevorzugt liegt in Schritt (B2) der Druckverlust über den Filter bei 0,01 bis 5 bar, bevorzugt bei 0,1 bis 3 bar.

Das in Schritt (B2) des erfindungsgemäßen Verfahrens erhaltene mindestens eine Isocyanat wird bevorzugt in Schritt (C) überführt. Bevorzugt betrifft die vorliegende Erfindung somit das erfindungsgemäße Verfahren, wobei die Schritte in der Reihenfolge (A), gefolgt von (B2), gefolgt von (C) durchgeführt werden.

Es ist erfindungsgemäß auch möglich, aber weniger bevorzugt, dass das in Schritt (A) bereitgestellte mindestens eine Isocyanat zunächst in Schritt (C) und anschließend in Schritt (B2) behandelt wird.

Schritt (C) des erfindungsgemäßen Verfahrens umfasst das Filtrieren des mindestens einen Isocyanates über einen Filter mit einer maximalen Porengröße von 0,02 bis 10 $\mu$m.

In Schritt (C) des erfindungsgemäßen Verfahrens wird das in Schritt (A) oder (B2) erhaltene mindestens eine Isocyanat über einen Filter mit einer maximalen Porengröße von 0,02 bis 10 $\mu$m, bevorzugt 0,05 bis 1 $\mu$m, besonders bevorzugt 0,1 bis 0,5 $\mu$m, ganz besonders bevorzugt 0,15 bis 0,3 $\mu$m, filtriert.

Im Rahmen der vorliegenden Erfindung wird die maximale Porengröße der eingesetzten Filter für flexible Filter, z. B. Membranfilter, nach ASTM F316-03 2011 und für starre Filter, z. B. Glas- oder Keramikfilter, nach ASTM E218-99 2011 bestimmt.

Erfindungsgemäß kann Schritt (C) bei jeder dem Fachmann als geeignet erscheinenden Temperatur erfolgen. Bevorzugt wird Schritt (C) bei einer Temperatur von 5 bis 190 °C, besonders bevorzugt 5 bis 100 °C, ganz besonders bevorzugt 5 bis 60 °C, durchgeführt.

Die vorliegende Erfindung betrifft daher bevorzugt das erfindungsgemäße Verfahren, wobei die Schritte (B1), (B2) und (C) jeweils bei einer Temperatur von 5 bis 190 °C durchgeführt werden.

Erfindungsgemäß kann Schritt (C) bei jedem dem Fachmann als geeignet erscheinenden Druck erfolgen. Bevorzugt wird Schritt (C) bei einem Druck von 0,1 bis 7 bar(a), besonders bevorzugt 0.5 bis 6 bar(a), ganz besonders bevorzugt 1 bis 4 bar(a), jeweils gemessen auf der Anströmseite des Filters, durchgeführt.

Abgesehen von der oben genannten maximalen Porengröße unterliegt der in Schritt (C) des erfindungsgemäßen Verfahrens eingesetzte Filter keinen weiteren Einschränkungen. Bevorzugt enthält der in Schritt (C) eingesetzte Filter mindestens ein Material ausgewählt aus Naturfasern, synthetischen Polymeren, Glas, Keramik, Metall und Mischungen davon.

Weiter bevorzugt wird in Schritt (C) erfindungsgemäß ein Filter, beispielsweise ein Kerzenfilter, Scheibenfilter, Platten- und Rahmenfilter, Schichtenfilter, Membrankartuschen, aus dem genannten Material eingesetzt. Geeignete Vorrichtungen zur Aufnahme der der entsprechenden Filter sind dem Fachmann an sich bekannt.

Weiter bevorzugt liegt in Schritt (C) des erfindungsgemäßen Verfahrens ein spezifischer Filtratfluss, von 20 bis 1000 kg/($m^2$·h), bevorzugt 50 bis 600 kg/($m^2$·h), besonders bevorzugt 100 bis 500 kg/($m^2$·h), vor.

Ebenfalls weiter bevorzugt liegt in Schritt (B1) der Druckverlust über den Filter bei 0,01 bis 5 bar, bevorzugt bei 0,1 bis 3 bar und besonders bevorzugt bei 0,5 bis 1,5 bar.

Die Filtrierschritte Schritt (B1), Schritt (B2) und Schritt (C) können unabhängig voneinander auf verschiedene Weisen ausgeführt werden.

Es ist beispielsweise möglich, den Filtrierschritt als sogenannte Dead-End-Filtration durchzuführen. Dabei wird

der zu filtrierende Fluid-Strom gegen den Filter gefördert und der einzige Auslass für das Fluid ist durch den Filter. Es bildet sich ein Filterkuchen oder zumindest ein Konzentrationsgradient aus den abzutrennenden Partikeln auf der stromaufwärtsgelegenen Seite des Filters aus, was den Filtrationswiderstand erhöht. Wird der Filtrationswiderstand zu hoch, kann durch eine Rückspülung mit dem Filtrat der Filterkuchen gelockert oder entfernt und der Filtrationswiderstand wieder gesenkt werden. Bevorzugt wird bei dieser Vorgehensweise der Druckunterschied gering gehalten, um eine Kompaktierung des Filterkuchens zu vermeiden.

Alternativ kann der zu filtrierende Fluid-Strom in einer Tangentialflussfiltration an dem Filter entlang geführt werden, d.h. es liegt mindestens ein Ein- und mindestens ein Auslass für das zu filtrierende Fluid auf der stromaufwärts gelegenen Seite des Filters vor. Je nach Abfluss-Richtung des Filtrats spricht man dann von einer Gegenstrom-Filtration, d.h. das Filtrat fließt in umgekehrter Richtung wie der Feed-Strom, einer Querstromfiltration, d.h. das Filtrat fließt senkrecht zum Feed-Strom, oder einer Gleichstromfiltration, d.h. das Filtrat fließt in gleicher Richtung wie der Feed-strom. Von einer Mixed-Flow-Filtration spricht man, wenn das zu filtrierende Fluid auf der stromaufwärts gelegenen Seite des Filters agitiert wird. Das Retentat kann entweder auf der stromaufwärts gelegenen Seite des Filters zirkuliert oder an anderer Stelle in den Prozess zurückgeführt werden. Das Verwerfen des Retentats ist aus wirtschaftlichen Gründen bei der Tangentialflussfiltration nicht empfehlenswert. Vorteilhaft an der Tangentialflussfiltration sind das Vermeiden eines Filterkuchens und das Minimieren des Filtrationswiderstands, jedoch erfordert das Fördern der Fluide einen höheren energetischen und apparativen Aufwand.

Bevorzugt erfolgen die Filtrierschritte Schritt (B1) bzw. Schritt (B2) und Schritt (C) als Dead-End-Filtration. Grund hierfür ist die hohe Energieeffizienz, da Kreislaufströme vermieden werden. Das Retentat kann letztlich bevorzugt zusammen mit dem Filtermaterial entsorgt werden.

In einer bevorzugten Ausführungsform des erfindungsgemäßen Verfahrens erfolgt die Filtration als Tiefenfiltration. Vorteilhaft an einer solchen Tiefenfiltration sind eine lange Standzeit des Filters einerseits und eine gute Abtrennung auch von schwer filtrierbaren Partikeln. Besonders bevorzugt besitzt dabei der Tiefenfilter eine asymmetrische Struktur, das heißt, die Poren des Filters werden von der Anströmseite her mit zunehmender Eindringtiefe in die Filterschicht feiner.

Nach Durchführen des Verfahrens umfassend die Schritte (A), gefolgt von (B2), gefolgt von (C) oder durch das Verfahren umfassend die Schritte (A), gefolgt von (C), gefolgt von (B2), wird erfindungsgemäß das mindestens eine gereinigte Isocyanat, bevorzugt Xylylendiisocyanat, insbesondere bevorzugt meta-Xylylendiisocyanat und/oder para-Xylylendiisocyanat, erhalten, welches eine Trübung von weniger als 0,35 NTU, besonders bevorzugt 0,05 bis 0,35 NTU, ganz besonders bevorzugt 0,10 bis 0,32 NTU, jeweils bestimmt nach DIN EN ISO 7027-1:2016, aufweist.

Durch diese erfindungsgemäß erhaltene besonders niedrige Trübung kann das mindestens eine gereinigte Isocyanat bevorzugt zur Synthese von Polyurethanen für optische Anwendungen eingesetzt werden.

Die vorliegende Erfindung betrifft des Weiteren ein Verfahren zur Herstellung mindestens eines Isocyanates ausgewählt aus der Gruppe bestehend aus aliphatischen, cycloaliphatischen, araliphatischen Isocyanaten und Mischungen davon, bevorzugt mindestens eines Diisocyanates ausgewählt aus der Gruppe bestehend aus aliphatischen, cycloaliphatischen, araliphatischen Diisocyanaten und Mischungen davon, umfassend mindestens die folgenden Schritte:

(A1) Bereitstellen mindestens eines dem herzustellenden Isocyanat entsprechenden Amins, bevorzugt Bereitstellen mindestens eines dem herzustellenden Diisocyanat entsprechenden Diamins,

(A2) Umsetzen des mindestens einen Amins aus Schritt (A1) mit Phosgen oder einem Derivat davon, um das mindestens eine Isocyanat zu erhalten, bevorzugt Umsetzen des mindestens einen Diamins aus Schritt (A1) mit Phosgen oder einem Derivat davon, um das mindestens eine Diisocyanat zu erhalten, und

(A3) Reinigung des mindestens einen Isocyanates aus Schritt (A2), bevorzugt Reinigung des mindesten einen Disocyanates aus Schritt (A2),

wobei Schritt (A3) mindestens den folgenden Schritt umfasst:
(B1) Filtrieren des mindestens einen Isocyanates aus Schritt (A2) über einen Filter, der eine Permeabilität von 5 bis 100 $1/(m^2 \cdot min)$, bei Standardbedingungen, $\Delta p$ = 1 bar, $H_2O$, 20 °C, aufweist, um das mindestens eine Isocyanat zu erhalten, bevorzugt Filtrieren des mindestens einen Diisocyanates aus Schritt (A2) über einen Filter, der eine Permeabilität von 5 bis 100 $1/(m^2 \cdot min)$ (bei Standardbedingungen, $\Delta p$ = 1 bar, $H_2O$, 20 °C) aufweist, um das mindestens eine Diisocyanat zu erhalten,
oder dass Schritt (A3) mindestens die folgenden Schritte umfasst:

(B2) Filtrieren des mindestens einen Isocyanates aus Schritt (A2) über einen Filter, der eine Permeabilität von 5 bis 1000 $1/(m^2 \cdot min)$ (bei Standardbedingungen, $\Delta p$ = 1 bar, $H_2O$, 20 °C) aufweist, bevorzugt Filtrieren des mindestens einen Diisocyanates aus Schritt (A2) über einen Filter, der eine Permeabilität von 5 bis 1000 $1/(m^2 \cdot min)$ (bei

Standardbedingungen, $\Delta p$ = 1 bar, $H_2O$, 20 °C) aufweist, und

(C) Filtrieren des mindestens einen Isocyanates über einen Filter mit einer maximalen Porengröße von 0,02 bis 10 $\mu$m, bevorzugt Filtrieren des mindestens einen Diisocyanates über einen Filter mit einer maximalen Porengröße von 0,02 bis 10 $\mu$m,

wobei die Reihenfolge der Schritte (A1), (A2) und (B1) oder (A1), (A2), (B2) und (C) oder (A1), (A2), (C) und (B2) sein kann, um das mindestens eine Isocyanat, bevorzugt das mindestens eine Diisocyanat, zu erhalten.

Schritt (A1) umfasst das Bereitstellen mindestens eines dem herzustellenden Isocyanat entsprechenden Amins. Erfindungsgemäß wird in Schritt (A1) demnach mindestens ein Amin ausgewählt aus der Gruppe bestehend aus aliphatischen, cycloaliphatischen, araliphatischen Aminen und Mischungen davon eingesetzt. Insbesondere bevorzugt wird in Schritt (A1) mindestens ein Diamin ausgewählt aus der Gruppe bestehend aus aliphatischen, cycloaliphatischen, araliphatischen Diaminen und Mischungen davon eingesetzt. Verfahren zur Herstellung entsprechender Amine und Diamine sind dem Fachmann an sich bekannt. Technisch relevante Verfahren sind beispielsweise die Hydrierung der entsprechenden Nitroverbindungen, die Hydrierung der entsprechenden Nitrile oder die reduktive Aminierung von Carbonylverbindungen.

Erfindungsgemäß geeignete und bevorzugte Diisocyanate sind weiter oben genannt und sollen auch für Schritt (A1) gelten.

Für den besonders bevorzugten Fall, dass erfindungsgemäß Bis(isocyanatomethyl)bicyclo[2.2.1]heptan (NBDI), insbesondere 2,5-Bis(isocyanatomethyl)bicyclo[2.2.1]heptan (2,5-NBDI) oder 2,6-Bis(isocyanatomethyl)bicyclo[2.2.1]heptan (2,6-NBDI), 1,5-Pentamethylendiisocyanat, 1,3-Bis(isocyanatomethyl)-cyclohexan (H6XDI), Xylylendiisocyanat, insbesondere meta-Xylylendiisocyanat und/oder para-Xylylendiisocyanat, oder Mischungen davon hergestellt werden, werden in Schritt (A1) bevorzugt Bis(aminomethyl)bicyclo[2.2.1]heptan (NBDA), insbesondere 2,5-Bis(aminomethyl)bicyclo[2.2.1]heptan (2,5-NBDA) oder 2,6-Bis(aminomethyl)bicyclo[2.2.1]heptan (2,6-NBDA), 1,5-Pentamethylendiamin, 1,3-Bis(aminomethyl)-cyclohexan (H6XDA), Xylylendiamin, insbesondere meta-Xylylendiamin und/oder para-Xylylendiamin, oder Mischungen davon eingesetzt.

Schritt (A2) des erfindungsgemäßen Verfahrens umfasst das Umsetzen des mindestens einen Amins aus Schritt (A1) mit Phosgen oder einem Derivat davon, um das mindestens eine Isocyanat zu erhalten.

Schritt (A2) des erfindungsgemäßen Verfahrens kann im Allgemeinen nach allen dem Fachmann bekannten Verfahren erfolgen, In einer bevorzugten Ausführungsform betrifft die vorliegende Erfindung das Verfahren, wobei Schritt (A2) in der Gasphase oder in der Flüssigphase, besonders bevorzugt in der Flüssigphase, erfolgt.

Insbesondere erfolgt die Phosgenierung des entsprechenden mindestens einen Amins erfindungsgemäß, indem das mindestens eine Amin in der flüssigen Phase mit Phosgen umgesetzt wird. Besonders geeignet sind zweistufige Phosgenier-Verfahren, wie die Kalt-Heiß-Phosgenierung, die Amin-Hydrochlorid-Phosgenierung oder die Carbaminat-Phosgenierung. Ganz besonders geeignet ist die Amin-Hydrochlorid-Phosgenierung, da sie auch bei sehr reaktiven Aminen wie beispielsweise XDA mit hoher Selektivität abläuft.

Die vorliegende Erfindung betrifft daher insbesondere das erfindungsgemäße Verfahren, wobei Schritt (A2) im Allgemeinen bei einer Temperatur von 5 bis 500 °C und/oder einem Druck von 0,5 bis 10 bar (a) erfolgt.

Erfolgt Schritt (A2), d.h. das Umsetzen des mindestens einen Amins aus Schritt (A1) mit Phosgen oder einem Derivat davon, um das mindestens eine Isocyanat zu erhalten, in der Gasphase, so liegt die Reaktionstemperatur vorzugsweise im Bereich von 300 bis 500 °C und der Druck im Bereich von 0,5 bis 3 bar(a).

Erfolgt Schritt (A2) als Basenphosgenierung in der flüssigen Phase, so wird zunächst eine Kaltphosgenierung, vorzugsweise bei einer Temperatur im Bereich von 0 bis 100 °C, besonders bevorzugt im Bereich von 10 bis 60 °C, und im Anschluss eine Heißphosgenierung bei erhöhter Temperatur, bevorzugt einer Temperatur im Bereich von 120 bis 200 °C durchgeführt. Der Druck liegt dabei vorzugsweise bei 1 bis 10 bar(a), besonders bevorzugt 1,2 bis 5 bar(a).

Erfolgt Schritt (A2) auf der Hydrochlorid- oder Carbaminatroute, so liegt die Temperatur während der Phsogenierungsreaktion vorzugsweise im Bereich von 80 bis 200 °C, besonders bevorzugt im Bereich von 120 bis 200 °C und ganz besonders bevorzugt im Bereich von 120 bis 180 °C. Der Druck liegt dabei vorzugsweise beil bis 10 bar(a), besonders bevorzugt 1,2 bis 5 bar(a).

Weitere Details und bevorzugte Ausführungsformen der Phosgenierung werden weiter oben beschrieben.

Schritt (A3) des erfindungsgemäßen Verfahrens umfasst die Reinigung des Isocyanates aus Schritt (A2), wobei Schritt (A3) mindestens den folgenden Schritt umfasst:

(B1) Filtrieren des mindestens einen Isocyanates aus Schritt (A2) über einen Filter, der eine Permeabilität von 5 bis 100 $1/(m^2 \cdot min)$ (bei Standardbedingungen, $\Delta p$ = 1 bar, $H_2O$, 20 °C) aufweist, um das mindestens eine Isocyanat zu erhalten, oder Schritt (A3) mindestens die folgenden Schritte umfasst:

(B2) Filtrieren des mindestens einen Isocyanates aus Schritt (A2) über einen Filter, der eine Permeabilität von 5 bis 1000 $1/(m^2 \cdot min)$ (bei Standardbedingungen, $\Delta p$ = 1 bar, $H_2O$, 20 °C) aufweist, und

(C) Filtrieren des mindestens einen Isocyanates über einen Filter mit einer maximalen Porengröße von 0,02 bis 10 µm,

wobei die Reihenfolge der Schritte (A1), (A2) und (B1) oder (A1), (A2), (B2) und (C) oder (A1), (A2), (C) und (B2) sein kann, um das mindestens eine Isocyanat zu erhalten.

Die Schritte (B1), (B2) und (C) werden weiter oben ausführlich beschrieben.

Die vorliegende Erfindung betrifft insbesondere das erfindungsgemäße Verfahren, wobei das mindestens eine Isocyanat in Schritt (A3) vor dem Filtrieren, d.h. vor den Schritten (B1) oder (B2) und (C) destilliert wird. Eine solche Destillation wird nach dem Fachmann bekannten Verfahren durchgeführt und die Reindestillation erfolgt beispielsweise bei einer Sumpftemperatur im Bereich von 120 bis 185 °C und einem Druck von 1 bis 100 mbar (a).

Durch das erfindungsgemäße Verfahren wird das mindestens eine Isocyanat mit einer sehr geringen Trübung erhalten. Die vorliegende Erfindung betrifft daher auch das mindestens eine Isocyanat, erhältlich, bevorzugt erhalten, durch das erfindungsgemäße Verfahren.

Weiterhin betrifft die vorliegende Erfindung auch ein Isocyanat, bevorzugt ein Diisocyanat, weiter bevorzugt Xylylendiisocyanat, insbesondere meta-Xylylendiisocyanat und/oder para-Xylylendiisocyanat, mit einer Trübung von weniger als 0,35 NTU, besonders bevorzugt 0,05 bis 0,35 NTU, ganz besonders bevorzugt 0,10 bis 0,32 NTU, jeweils bestimmt nach DIN EN ISO 7027-1:2016.

Die vorliegende Erfindung betrifft auch ein Polymer, aufgebaut aus mindestens einem erfindungsgemäßen Disocyanat und mindestens einer gegenüber Isocyanat reaktiven Verbindung ausgewählt aus der Gruppe bestehend aus Polythiolen enthaltend mindestens zwei Thiolgruppen, Hydroxythiolen enthaltend je mindestens eine Hydroxy- und Thiolgruppe, und Polyolen enthaltend mindestens zwei Hydroxygruppen. Es ist auch möglich Mischungen verschiedener gegenüber Isocyanat reaktiver Verbindungen einzusetzen. Bevorzugt ist die gegenüber Isocyanat reakive Verbindung ein Polythiol oder eine Mischung aus zwei oder mehr Polythiolen.

Als Polythiole eignen sich beispielsweise Methandithiol, 1,2-Ethandithiol, 1,1-Propandithiol, 1,2-Propandithiol, 1,3-Propandithiol, 2,2-Propandithiol, 1,4-Butandithiol, 2,3-Butandithiol, 1,5-Pentandithiol, 1,6-Hexandithiol, 1,2,3-Propantrithiol, 1,1-Cyclohexandithiol, 1,2-Cyclohexandithiol, 2,2-Dimethylpropan-1,3-dithiol, 3,4-Dimethoxybutan-1,2-dithiol und 2-Methylcyclohexan-2,3-dithiol, Thioethergruppen enthaltende Polythiole, wie z. B. 2,4-Dimercaptomethyl-1,5-dimercapto-3-thiapentan, 4-Mercaptomethyl-1,8-dimercapto-3,6-dithiaoctan, , 4,8-Dimercaptomethyl-1,11-dimercapto-3,6,9-trithiaundecan, 4,7-Dimercaptomethyl-1,11-dimercapto-3,6,9-trithiaundecan, 5,7-Dimercaptomethyl-1,11-dimercapto-3,6,9-trithiaundecan, 4,5-Bis(mercaptoethylthio)-1,10-dimercapto-3,8-dithiadecan, Tetrakis(mercaptomethyl)methan, 1,1,3,3-Tetrakis(mercaptomethylthio)propan, 1,1,5,5-Tetrakis(mercaptomethylthio)-3-thiapentan, 1,1,6,6-Tetrakis(mercaptomethylthio)-3,4-dithiahexan, 2-Mercaptoethylthio-1,3-dimercaptopropan, 2,3-Bis(mercaptoethylthio)-1-mercaptopropan, 2,2-Bis(mercaptomethyl)-1,3-dimercaptopropan, Bis(mercaptomethyl)sulfid, Bis(mercaptomethyl)disulfid, Bis(mercaptoethyl)sulfid, Bis(mercaptoethyl)disulfid, Bis(mercaptopropyl)sulfid, Bis(mercaptopropyl)disulfid, Bis(mercaptomethylthio)methan, Tris(mercaptomethylthio)methan, Bis(mercaptoethylthio)methan, Tris(mercaptoethylthio)methan, Bis(mercaptopropylthio)methan, 1,2-Bis(mercaptomethylthio)ethan, 1,2-Bis(mercaptoethylthio)ethan, 2-Mercaptoethylthio)ethan, 1,3-Bis(mercaptomethylthio)propan, 1,3-Bis(mercaptopropylthio)propan, 1,2,3-Tris(mercaptomethylthio)propan, 1,2,3-Tris(mercaptoethylthio)propan, 1,2,3-Tris(mercaptopropylthio)propan, Tetrakis(mercaptomethylthio)methan, Tetrakis(mercaptoethylthiomethyl)methan, Tetrakis(mercaptopropylthiomethyl)methan, 2,5-Dimercapto-1,4-dithian, 2,5-Bis(mercaptomethyl)-1,4-dithian und dessen gemäß JP-A 07118263 erhältliche Oligomere, 1,5-Bis(mercaptopropyl)-1,4-dithian, 1,5-Bis(2-mercaptoethylthiomethyl)-1,4-dithian, 2-Mercaptomethyl-6-mercapto-1,4-dithiacycloheptan, 2,4,6-Trimercapto-1,3,5-trithian, 2,4,6-Trimercaptomethyl-1,3,5-trithian und 2-(3-Bis(mercaptomethyl)-2-thiapropyl)-1,3-dithiolan, Polyesterthiole, wie z. B. Ethylenglycol-bis(2-mercaptoacetat), Ethylenglycol-bis(3-mercaptopropionat), Diethylenglycol(2-mercaptoacetat), Diethylenglycol(3-mercaptopropionat), 2,3-Dimercapto-1-propanol(3-mercaptopropionat), 3-Mercapto-1,2-propandiol-bis(2-mercaptoacetat), 3-Mercapto-1,2-propandiol-bis(3-mercaptopropionat), Trimethylolpropan-tris(2-mercaptoacetat), Trimethylolpropan-tris(3-mercaptopropionat), Trimethylolethan-tris(2-mercaptoacetat), Trimethylolethan-tris(3-mercaptopropionat), Pentaerythrit-tetrakis(2-mercaptoacetat), Pentaerythrit-tetrakis(3-mercaptopropionat), Glycerin-tris(2-mercaptoacetat), Glycerin-tris(3-mercaptopropionat), 1,4-Cyclohexandiol-bis(2-mercaptoacetat), 1,4-Cyclohexandiol-bis(3-mercaptopropionat), Hydroxymethylsulfid-bis(2-mercaptoacetat), Hydroxymethylsulfid-bis(3-mercaptopropionat), Hydroxyethylsulfid(2-mercaptoacetat), Hydroxyethylsulfid(3-mercaptopropionat), Hydroxymethyldisulfid(2-mercaptoacetat), Hydroxymethyldisulfid(3-mercaptopropionat), (2-Mercaptoethylester)thioglycolat und Bis(2-mercaptoethylester)thiodipropionat sowie aromatische Thioverbindungen, wie z. B. 1,2-Dimercaptobenzol, 1,3-Dimercaptobenzol, 1,4-Dimercaptobenzol, 1,2-Bis(mercaptomethyl)benzol, 1,4-Bis(mercaptomethyl)benzol, 1,2-Bis(mercaptoethyl)benzol, 1,4-Bis(mercaptoethyl)benzol, 1,2,3-Trimercaptobenzol, 1,2,4-Trimercaptobenzol, 1,3,5-Trimercaptobenzol, 1,2,3-Tris(mercaptomethyl)benzol, 1,2,4-Tris(mercaptomethyl)benzol, 1,3,5-Tris(mercaptomethyl)benzol, 1,2,3-Tris(mercaptoethyl)benzol, 1,3,5-Tris(mercaptoethyl)benzol, 1,2,4-Tris(mercaptoethyl)benzol, 2,5-Toluoldithiol, 3,4-Toluoldithiol, 1,4-Naphthalindithiol, 1,5-Naphthalindithiol, 2,6-Naphthalindithiol, 2,7-Naphthalindithiol, 1,2,3,4-Tetramercaptobenzol, 1,2,3,5-Tetramercaptobenzol, 1,2,4,5-Tetramercaptobenzol, 1,2,3,4-Tetrakis(mercaptomethyl)benzol, 1,2,3,5-Tetrakis(mercaptomethyl)benzol, 1,2,4,5-Tetrakis(mercaptomethyl)benzol, 1,2,3,4-Tetrakis(mercaptoethyl)benzol, 1,2,3,5-Tetrakis(mercaptoethyl)benzol, 1,2,4,5-Tetrakis(mercaptoethyl)benzol, 2,2'-Dimercaptobiphenyl, 4,4'-Dimercaptobiphenyl oder Mischungen davon.

Vorzugsweise ist das Polythiol ausgewählt aus 4-Mercaptomethyl-1,8-dimercapto-3,6-dithiaoctan, , 2,5-Bismercaptomethyl-1,4-dithian, 1,1,3,3-Tetrakis(mercaptomethylthio)propan, 5,7-Dimercaptomethyl-1,11-dimercapto-3,6,9-trithiaundecan, 4,7-Dimercaptomethyl-1,11-dimercapto-3,6,9-trithiaundecan, 4,8-Dimercaptomethyl-1,11-dimercapto-3,6,9-trithiaundecan, Trimethylolpropan-tris(3-mercaptopropionat), Trimethylolethan-tris(2-mercaptoacetat), Pentaerythrit-tetrakis(2-mercaptoacetat), Pentaerythrit-tetrakis(3-mercaptopropionat) oder Mischungen davon.

Abgesehen von der Thiolkomponente B) kann die erfindungsgemäße Zusammensetzung auch andere, üblicherweise mit Polyisocyanaten reagierende Komponenten enthalten. Hierbei handelt es sich insbesondere um die üblichen aus der Polyurethanchemie bekannten Polyetherpolyole, Polyesterpolyole, Polyetherpolyesterpolyole, Polythioetherpolyole, polymermodifizierte Polyetherpolyole, Pfropfpolyetherpolyole, insbesondere solche auf Styrol- und/oder Acrylnitrilbasis, Polyetherpolyamine, hydroxylgruppenhaltigen Polyacetale und/oder hydroxylgruppenhaltigen aliphatischen Polycarbonate, die üblicherweise ein gewichtsmittleres Molekulargewicht von 106 bis 12000 g/mol aufweisen, vorzugsweise 250 bis 8000 g/mol. Ein breiter Überblick über geeignete Reaktionspartner B) findet sich beispielsweise in N. Adam et al.: "Polyurethanes", Ullmann's Encyclopedia of Industrial Chemistry, Electronic Release, 7th ed., chap. 3.2 - 3.4, Wiley-VCH, Weinheim 2005.

Die vorliegende Erfindung betrifft auch die Verwendung des erfindungsgemäßen Isocyanates zur Herstellung von Polyurethanen für optische Bauteile. Beispiele für optische Bauteile sind beispielsweise optische Linsen, Brillengläser oder optoelektronische Bauteile wie Leuchtdioden.

Die vorliegende Erfindung betrifft daher des Weiteren auch ein optisches Bauteil, bevorzugt eine Linse oder ein Brillenglas, enthaltend mindestens ein erfindungsgemäßes Polyurethan.

**Beispiele:**

Unter der Permeabilität der eingesetzten Filter ist erfindungsgemäß die Menge an reinem Wasser zu verstehen, die bei Standard-Bedingungen, also 20 °C und 1 bar Druckdifferenz die Filterschicht je Zeiteinheit und Fläche passiert. Zur Bestimmung der Permeabilität wird Reinstwasser verwendet. Der Filter oder eine repräsentative Probe des Filtermaterials wird bei 20 °C mit Reinstwasser beaufschlagt und die gewünschte Druckdifferenz von 1 bar zwischen der Anströmseite und der Filtratseite eingestellt. Es werden die Zeit und die in dieser Zeit anfallende Filtratmenge gemessen, woraus dann unter Berücksichtigung der bekannten Fläche des Filters die Permeabilität gemäß folgender Formel berechnet werden kann:

$$\text{Permeabilität} = \text{Filtratmenge in Litern} / (\text{Filterfläche in } m^2 \text{ x Zeit in min})$$

Eine geeignete Vorrichtung für die Messung ist beispielsweise in der VDI-Richtlinie 2762, Blatt 2, Seiten 6-8 beschrieben. Relevant für das erfindungsgemäße Verfahren ist der Anfangswert der Permeabilität des eingesetzten Filters vor der Verwendung.

Die maximale Porengröße der eingesetzten Filter wird flexible Filter, z.B. Membranfilter, nach ASTM F316-03 2011 und für starre Filter, z.B. Glas- oder Keramikfilter, nach ASTM E218-99 2011 bestimmt.

Die Trübung der Isocyanate wird bestimmt nach DIN EN ISO 7027-1:2016-11, wobei das Isocyanat entsprechend dem wässrigen Medium gemäß dieser DIN behandelt wird.

Ausgehend von meta-Xylylendiamin (meta-XDA) wird durch Umsetzung mit Phosgen in der Gasphase meta-Xylylendiisocyanat (meta-XDI) erhalten. Das Rohprodukt wird durch Destillation bei 170 °C und einem Druck von 15 mbar (a) aufgereinigt. Das so erhaltene meta-XDI ("Ausgangslösung", Versuch 9 in Tabelle 1) weist eine Trübung von 25,4 NTU, bestimmt nach DIN EN ISO 7027-1:2016-11, auf. Diese Ausgangslösung wird gemäß den in Tabelle 1 genannten Versuchen 1, 2, 3, 4, 5, V6, 7 und V8 filtriert.

Die Ergebnisse sind in Tabelle 1 dargestellt. In den erfindungsgemäßen Versuchen 1, 2, 3, 4, 5 und 7 werden erfindungsgemäß gewünschte Trübungswerte von $\leq$ 0,35 NTU erhalten, so dass Polyurethane, die mit diesem meta-XDI hergestellt werden, die hohen Anforderungen für die Verwendung in optischen Linsen erfüllen, während in den Vergleichsversuchen V6 und V8 unvorteilhafte hohe Trübungswerte erhalten werden, so dass daraus hergestellte Polyurethane nicht für die Anwendung in optischen Linsen geeignet sind.

Tabelle 1:

| Nummer | 1 | 2 | 3 | 4 | 5 | V6 | 7 | V8 | 9 Ausgangslösung |
|---|---|---|---|---|---|---|---|---|---|
| **Permeabilität [l/m$^2$ · min] in der 1. Filtrationsstufe** | 29 | 29 | 100 | 100 | 146 | 146 | 925 | 925 | - |
| **Maximale Porengröße [µm] in der 2. Filtrationsstufe** | - | 0,2 | 0,2 | 0,2 | 0,2 | - | 0,2 | - | - |
| **Trübung [NTU][1]** | 0,27 | 0,23 | 0,32 | 0,29 | 0,26 | 14,7 | 0,32 | 20,8 | 25,4 |

V Vergleichsversuch
- nicht durchgeführt
[1] bestimmt nach DIN EN ISO 7027-1:2016

**Patentansprüche**

1. Verfahren zur Reinigung mindestens eines Isocyanates ausgewählt aus der Gruppe bestehend aus aliphatischen, cycloaliphatischen, araliphatischen Isocyanaten und Mischungen davon, umfassend mindestens die folgenden Schritte:

   (A) Bereitstellen des mindestens einen Isocyanates,
   (B1) Filtrieren des mindestens einen Isocyanates aus Schritt (A) über einen Filter, der eine Permeabilität von 5 bis 100 1/(m$^2$ · min) (bei Standardbedingungen, $\Delta p$ = 1 bar, $H_2O$, 20 °C) aufweist, um das mindestens eine gereinigte Isocyanat zu erhalten,

   oder umfassend mindestens die folgenden Schritte:

   (A) Bereitstellen des mindestens einen Isocyanates,
   (B2) Filtrieren des mindestens einen Isocyanates aus Schritt (A) über einen Filter, der eine Permeabilität von 5 bis 1000 1/(m$^2$ · min) (bei Standardbedingungen, $\Delta p$ = 1 bar, $H_2O$, 20 °C) aufweist, und
   (C) Filtrieren des mindestens einen Isocyanates über einen Filter mit einer maximalen Porengröße von 0,02 bis 10 µm,

   wobei die Reihenfolge der Schritte (A), (B2), (C) oder (A), (C), (B2) sein kann, um das mindestens eine gereinigte Isocyanat zu erhalten.

2. Verfahren nach Anspruch 1, **dadurch gekennzeichnet, dass** das mindestens eine gereinigte Isocyanat zur Synthese von Polyurethanen für optische Anwendungen eingesetzt wird.

3. Verfahren nach Anspruch 1 oder 2, **dadurch gekennzeichnet, dass** das mindestens eine Isocyanat ausgewählt ist aus der Gruppe bestehend aus Bis(isocyanatomethyl)bicyclo[2.2.1]heptan (NBDI), insbesondere 2,5-Bis(isocyanatomethyl)bicyclo[2.2.1]heptan (2,5-NBDI) oder 2,6-Bis(isocyanatomethyl)bicyclo[2.2.1]heptan (2,6-NBDI), Pentamethylendiisocyanat, 1,3-Bis(isocyanatomethyl)cyclohexan (H6XDI), Xylylendiisocyanat, insbesondere meta-Xylylendiisocyanat und/oder para-Xylylendiisocyanat, und Mischungen davon.

4. Verfahren nach einem der Ansprüche 1 bis 3, **dadurch gekennzeichnet, dass** das mindestens eine gereinigte Isocyanat eine Trübung von ≤ 0,35 NTU, bestimmt nach DIN EN ISO 7027-1:2016, aufweist.

5. Verfahren nach einem der Ansprüche 1 bis 4, **dadurch gekennzeichnet, dass** Schritt (A) das Destillieren des mindestens einen Isocyanates, insbesondere vor den Schritten (B1) oder (B2), umfasst.

6. Verfahren nach einem der Ansprüche 1 bis 5, **dadurch gekennzeichnet, dass** die in Schritt (B1) oder (B2) eingesetzte Filterschicht mindestens ein Material ausgewählt aus Naturfasern, synthetischen Polymeren, Perlit, Kieselgur und Mischungen davon, enthält.

7. Verfahren nach einem der Ansprüche 1 bis 6, **dadurch gekennzeichnet, dass** in Schritt (B1) und/oder (B2) ein spezifischer Filtratfluss, bei Druckverlust von 1 bar am Filter von 50 bis 1000 kg/($m^2 \cdot h$), bevorzugt 500 bis 800 kg/($m^2 \cdot h$), vorliegt.

8. Verfahren zur Herstellung mindestens eines Isocyanates ausgewählt aus der Gruppe bestehend aus aliphatischen, cycloaliphatischen, araliphatischen Isocyanaten und Mischungen davon, umfassend mindestens die folgenden Schritte:

   (A1) Bereitstellen mindestens eines dem herzustellenden Isocyanat entsprechenden Amins,
   (A2) Umsetzen des mindestens einen Amins aus Schritt (A1) mit Phosgen oder einem Derivat davon, um das mindestens eine Isocyanat zu erhalten, und
   (A3) Reinigung des mindestens einen Isocyanates aus Schritt (A2),

   **dadurch gekennzeichnet, dass** Schritt (A3) mindestens den folgenden Schritt umfasst:
   (B1) Filtrieren des mindestens einen Isocyanates aus Schritt (A2) über einen Filter, der eine Permeabilität von 5 bis 100 1/($m^2 \cdot$ min) (bei Standardbedingungen, $\Delta p$ = 1 bar, $H_2O$, 20 °C) aufweist, um das mindestens eine Isocyanat zu erhalten,
   oder dass Schritt (A3) mindestens die folgenden Schritte umfasst:

   (B2) Filtrieren des mindestens einen Isocyanates aus Schritt (A2) über einen Filter, der eine Permeabilität von 5 bis 1000 1/($m^2 \cdot$ min) (bei Standardbedingungen, $\Delta p$ = 1 bar, $H_2O$, 20 °C) aufweist, und
   (C) Filtrieren des mindestens einen Isocyanates über einen Filter mit einer maximalen Porengröße von 0,02 bis 10 $\mu$m,

   wobei die Reihenfolge der Schritte (A1), (A2) und (B1) oder (A1), (A2), (B2) und (C) oder (A1), (A2), (C) und (B2) sein kann, um das mindestens eine Isocyanat zu erhalten.

9. Verfahren nach Anspruch 8, **dadurch gekennzeichnet, dass** Schritt (A2) bei einer Temperatur von 5 bis 500 °C und/oder einem Druck von 0,5 bis 10 bar (a) erfolgt.

10. Verfahren nach Anspruch 8 oder 9, **dadurch gekennzeichnet, dass** das mindestens eine Isocyanat in Schritt (A3) vor dem Filtrieren destilliert wird.

11. Isocyanat, erhältlich, bevorzugt erhalten, durch das Verfahren gemäß einem der Ansprüche 1 bis 10.

12. Isocyanat, bevorzugt ein Diisocyanat, weiter bevorzugt Xylylendiisocyanat, insbesondere meta-Xylylendiisocyanat und/oder para-Xylylendiisocyanat, **dadurch gekennzeichnet, dass** es eine Trübung von weniger als 0,35 NTU, besonders bevorzugt 0,05 bis 0,35 NTU, ganz besonders bevorzugt 0,10 bis 0,32 NTU, jeweils bestimmt nach DIN EN ISO 7027-1:2016 aufweist.

13. Polyurethan, aufgebaut aus mindestens einem Isocyanat gemäß Anspruch 11 oder 12 und mindestens einem Polyol.

14. Verwendung des Isocyanates nach Anspruch 11 oder 12 zur Herstellung von Polyurethanen für optische Anwendungen.

15. Optisches Bauteil, enthaltend mindestens ein Polyurethan gemäß Anspruch 13.

Europäisches Patentamt
European Patent Office
Office européen des brevets

# EUROPÄISCHER RECHERCHENBERICHT

Nummer der Anmeldung
EP 19 17 2870

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X<br><br><br>Y | CN 109 369 457 A (HUNAN HAILI CHANGDE PESTICIDE & CHEMICAL IND CO LTD)<br>22. Februar 2019 (2019-02-22)<br>* Seite 1, Absatz [0002]; Ansprüche; Beispiele * | 11-15<br><br><br>1-10 | INV.<br>C07C263/20<br>C07C265/14 |
| Y | US 3 522 285 A (KIRSS VOLDEMAR)<br>28. Juli 1970 (1970-07-28)<br>* Zusammenfassung; Ansprüche; Beispiel 1 *<br>* Spalte 1, Zeile 46 - Spalte 2, Zeile 23 * | 1-15 | |
| Y | US 3 575 820 A (SCHNABEL WILHELM J ET AL)<br>20. April 1971 (1971-04-20)<br>* Zusammenfassung *<br>* Spalte 1, Zeile 50 - Spalte 2, Zeile 49 * | 1-15 | |
| Y | GB 1 353 787 A (ICI LTD)<br>22. Mai 1974 (1974-05-22)<br>* Spalte 2, Zeilen 54-70; Beispiel 1 * | 1-15 | RECHERCHIERTE SACHGEBIETE (IPC)<br>C07C |

Der vorliegende Recherchenbericht wurde für alle Patentansprüche erstellt

| Recherchenort | Abschlußdatum der Recherche | Prüfer |
|---|---|---|
| München | 16. Oktober 2019 | Kiernan, Andrea |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

1

## ANHANG ZUM EUROPÄISCHEN RECHERCHENBERICHT ÜBER DIE EUROPÄISCHE PATENTANMELDUNG NR. EP 19 17 2870

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

16-10-2019

| Im Recherchenbericht angeführtes Patentdokument | Datum der Veröffentlichung | Mitglied(er) der Patentfamilie | Datum der Veröffentlichung |
|---|---|---|---|
| CN 109369457 A | 22-02-2019 | KEINE | |
| US 3522285 A | 28-07-1970 | BE 631132 A | 16-10-2019 |
| | | BE 681132 A | 31-10-1966 |
| | | DE 1568011 A1 | 30-04-1970 |
| | | GB 1133668 A | 13-11-1968 |
| | | US 3420752 A | 07-01-1969 |
| | | US 3522285 A | 28-07-1970 |
| US 3575820 A | 20-04-1971 | KEINE | |
| GB 1353787 A | 22-05-1974 | DE 2246920 A1 | 05-04-1973 |
| | | FR 2155362 A5 | 18-05-1973 |
| | | GB 1353787 A | 22-05-1974 |
| | | JP S4840719 A | 15-06-1973 |
| | | NL 7213043 A | 29-03-1973 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 1805957
- US 3658656 A
- GB 1413074 A
- WO 2007051740 A1
- JP 07118263 A


### In der Beschreibung aufgeführte Nicht-Patentliteratur


- **W. SIEFKEN.** *Liebigs Annalen der Chemie,* 1949, vol. 562, 96